# EUROPÄISCHE PATENTSCHRIFT

(11) **EP 1 987 707 B1**
(45) Veröffentlichungstag und Bekanntmachung des Hinweises auf die Patenterteilung: **29.07.2009**
(21) Anmeldenummer: 07703221.7
(22) Anmeldetag: 02.02.2007
(51) Int. Cl.: H05K 3/18, H01L 21/48, C23C 18/16

(54) **VERFAHREN ZUR HERSTELLUNG EINER SCHICHT AUF EINEM FORMKÖRPER**
METHOD FOR PRODUCING A LAYER ON A MOULDING
PROCÉDÉ DE PRODUCTION D'UNE COUCHE SUR UN CORPS FAÇONNÉ

(30) Priorität: 17.02.2006 DE 102006007397
(43) Veröffentlichungstag der Anmeldung: 05.11.2008
(73) Patentinhaber: Forschungszentrum Karlsruhe GmbH, 76133 Karlsruhe (DE)
(72) Erfinder: DAMBROWSKY, Nina, 10553 Berlin (DE); GISELBRECHT, Stefan, 76187 Karlsruhe (DE); TRUCKENMÜLLER, Roman, 74223 Flein (DE)
(74) Vertreter: Gärtner, Stephan
(86) Internationale Anmeldenummer: PCT/EP2007/000892
(87) Internationale Veröffentlichungsnummer: WO 2007/093290

(56) Entgegenhaltungen:
- EP-A1- 0 641 152
- EP-A1- 1 191 127
- EP-A1- 1 274 288
- REINHARD STRANSKY: "New 3D-MID process set to boost mechatronic applications Ultramid T as carrier for electrical circuits: MID and laser direct structuring" BASF NEWS RELEASE : TRADE PRESS CONFERENCE K 2004, [Online] 22. Juni 2004 (2004-06-22), - 23. Juni 2004 (2004-06-23) XP002449662 Gefunden im Internet: URL:http://www.corporate.basf.com/en/press e/konferenzen/k2004/vortrag-III-4.htm?geta sset=file2&name=III-4-P253e.pdf&MTITEL=Pre sentation+with+Charts%3Cbr%3E(PDF-file,+66 4+KB)&suffix=.pdf&id=kslUhB6inbcp*lE>

## Beschreibung

Die Erfindung betrifft ein Verfahren zur Herstellung einer vorzugsweise elektrisch leitfähigen Schicht auf einem Formkörper, der durch Umformen einer Folie gebildet wird.

Das Warm(um)formen oder Thermoformen von dünnen, am Rand fest eingespannten thermoplastischen Folien und Platten zu dreidimensionalen Formkörpern unter Ausdünnung ihrer Wanddicke ist neben dem Spritzguss das im makroskopischen Bereich wichtigste Verfahren zur Fertigung großer Stückzahlen an Kunststoffartikeln. Im Gegensatz zum Spritzguss ermöglicht dieses Verfahren auch im mikrotechnischen Bereich, dünnwandige und großflächige, dreidimensionale mikrostrukturierte Teile bzw. Mikrostrukturen herzustellen.

Leiterbahnen auf dreidimensionalen spritzgegossenen oder heißgeprägten Kunststoffteilen werden heutzutage vorwiegend durch die so genannte MID-Technologie (*moulded interconnect devices*) erzeugt. Typische Varianten dieses Verfahrens sind z.B. Zweikomponentenspritzgießen, Heißprägen, Maskenbelichtungsverfahren, Laserdirektstrukturierung und Folienhinterspritzen. Mit den genannten Verfahren lassen sich zwar dreidimensionale Strukturen mit integrierten Leiterbahnen erzeugen, jedoch können damit keine allseitig dünnwandigen und membranartigen dreidimensionalen Mikrostrukturen, insbesondere nicht mit Hinterschnitten hergestellt werden.

Beim Einsatz der bereits bestehenden MID-Verfahren besteht das Problem, dass zwar Leiterbahnen entweder nur mit hohem Aufwand in dreidimensionale Strukturen eingebracht werden können oder nur mit Breiten größer als einige 10 µm. Damit ist die Gestaltungsfreiheit erheblich eingeschränkt, insbesondere wenn es sich um Strukturen im mikroskopischen Maßstab handelt.

Hierbei stellt das Zweikomponentenspritzgießen das Verfahren mit den größten geometrischen Freiheiten unter den MID-Verfahren dar. Die kleinste realisierbare Leiterbahnbreite liegt in Abhängigkeit von den Fließeigenschaften und den Fließlängen im Werkzeug jedoch nur in einem Größenbereich von etwa 250 µm [Krautheim, T. B., Herstellungsverfahren, Gebrauchsanforderungen und Materialkennwerte Räumlicher Elektronischer Baugruppen 3-D MID: Handbuch für Anwender und Hersteller, Forschungsvereinigung Räumliche Elektronische Baugruppen, 2. Ausg., 1999].

Mit anderen Verfahren wie z.B. dem Heißprägen lassen sich zwar geringere Leiterbahngeometrien (ca. 125 µm) realisieren, allerdings bestehen hier deutliche Einschränkungen in Bezug auf die Gestaltung der Formkörper. Im Falle von Laserstrukturierung können teilweise (z.B. in semi-additiven Verfahren) Geometrien bis etwa 30 µm erzielt werden, jedoch erfordern hier komplexe Bauteilgeometrien einen hohen systemtechnischen Arbeitsaufwand (Mehrachs-Anlagen, 3D Eokuslagensteuerung).

Resistbasierte Laserstrukturierungsverfahren erleiden in der Regel den Nachteil, dass eine homogene Beschichtung von dreidimensionalen Bauteilen nur bei vergleichsweise einfachen Geometrien erzielt werden kann. Der hohe wirtschaftliche Einsatz dieses Verfahrens rechnet sich daher bisher nur im Bereich der Feinleiterstrukturierung [Krautheim, T. B., Herstellungsverfahren, Gebrauchsanforderungen und Materialkennwerte Räumlicher Elektronischer Baugruppen 3-D MID: Handbuch für Anwender und Hersteller, Forschungsvereinigung Räumliche Elektronische Baugruppen, 2. Ausg., 1999].

Maskenbasierte Verfahren zur Laserstrukturierung von Leiterbahnen haben entweder die typischen Nachteile einer Maskenprojektion oder es müssen aufwendige dreidimensionale Masken für die Belichtung hergestellt werden. Das Kontaktmaskenverfahren eignet sich daher auch nur für relativ einfache Bauteilgeometrien und vorzugsweise für größere Bauteile, da im mikroskopischen Bereich noch ein erheblicher Mehraufwand hinsichtlich der genauen Positionierung erforderlich wird. Im Fall des Folienhinterspritzens ist die Dreidimensionalität der Strukturen aufgrund des Auftretens von Falten bei der Umformung sehr stark eingeschränkt.

Aufgabe der Erfindung ist es, ein Verfahren zur Herstellung einer Schicht auf einem Formkörper bereitzustellen, die vorzugsweise elektrisch leitfähig ist, und womit es daher möglich wird, sehr schmale Leiterbahnen oder Elektroden auf dreidimensionale dünnwandige Mikrostrukturen aufzubringen.

Gelöst wird diese Aufgabe durch die Schritte des Verfahrens gemäß Patentanspruch 1. Die übrigen Ansprüche beschreiben vorteilhafte Ausgestaltungen der Erfindung.

Das erfindungsgemäße Verfahren zur Herstellung einer Schicht zumindest auf ausgewählten Bereichen auf der Oberfläche eines Formkörpers umfasst die Schritte a) bis d).

Im ersten Schritt a) wird eine umformbare Folie bereitgestellt, die vorzugsweise aus Kunststoff, besonders bevorzugt aus einem thermoplastischen Kunststoff, insbesondere Polymethylmethacrylat PMMA, Polyethylen PE, Polypropylen PP, Polycarbonat PC, Polystyrol PS, Polyamid PA, Polyethylenterephthalat PET, Polyimid PI, Polyvinyl(iden)chlorid PV(D)C, Polyvinyl(iden)fluorid PV(D)F oder Cycloolefincopolymer (COC) besteht. Vorzugsweise weist die Folie, die zwei-oder mehrlagig sein kann, eine Dicke von 1 bis 1000 µm, besonders bevorzugt von 5 bis 200 µm, insbesondere von 20 bis 50 µm auf.

Die Oberfläche des bereitgestellten Kunststoffs wird anschließend gemäß Schritt b) in ausgewählten (Teil-)Bereichen aktiviert. Dazu wird ein Verfahren herangezogen, das es erlaubt, in den ausgewählten Bereichen punktuell (lokal) galvanisch katalytisch wirksame Keime aufzubringen oder zu erzeugen.

Verfahren, die sich hierfür einsetzen lassen, verwenden Licht- oder Teilchenstrahlung wie PVD/CVD-Prozesse, laser- oder elektronenstrahlgestützte Verfahren [Shafeev, G. A. & Hoffmann,P., Lightenhanced electroless Cu deposition on laser-treated polyimide surface, Appl. Surface Sci. 138-139, 455-460 (1999); Kordas, K., Leppavuori, S., Uusimaki, A., George, T. F., Nanai, L., Vajtai, R., Bali, K. & Bekesi, J., Palladium thin film deposition on polyimide by CW Ar+ laser radiation for electroless copper plating, Thin Solid Films 384, 185-188 (2001); Cicoira, F., Hoffmann, P., Olsson, C. O. A., Xanthopoulos, N., Mathieu, H. J. & Doppelt, P., Auger electron spectroscopy analysis of high metal content micro-structures grown by electron beam induced deposition, Appl. Surface Sci. 242, 107-113 (2005) und Utke, I., Dwir, B., Leifer, K., Cicoira, F., Doppelt, P., Hoffmann, P. & Kapon, E., Electron beam induced deposition of metallic tips and wires for microelectronics applications, Microelectr. Engineering 53, 261-264 (2000)] oder Rasterstrukturierungsverfahren. Da es sich beim Ausgangsmaterial umformbare Folie um ein planares Substrat handelt, kann eine hochauflösencle und einfache Direktstrukturierung oder alternativ eine maskenbasierte Strukturierung durchgeführt werden.

Im nächsten Schritt c) wird die in ausgewählten (Teil-)Bereichen mit Keimen versehene (bekeimte) Folie in einem Umformverfahren, bevorzugt einem Thermoformverfahren, zu einem dreidimensionalen Formkörper umgeformt, vorzugsweise verstreckt. Dadurch dass die Folie während und nach diesem Verfahrensschritt jedoch nie ihren materiellen Zusammenhalt verliert und höchstens in einer gummielastischen Phase verstreckt wird, bleibt die Struktur der aktivierten Flächen erhalten, auch wenn diese entsprechend der Verstreckung verzerrt werden.

Im Unterschied zur Reihenfolge dieses Vorgehens wäre es nachteilig, bereits auf der Folie eine geschlossene Schicht zu erzeugen. Eine derartige geschlossene Schicht würde in der Regel bei Verstreckung versagen, insbesondere dann, wenn es sich um eine metallische Schicht mit einer deutlich höheren Neigung zur Rissbildung als die der Folie handelt. In diesem Fall würden sich Risse bilden, die unter Umständen so groß sind, dass sie nicht mehr mit einer dann erforderlichen außenstromlosen Galvanik überbrückbar sind oder geschlossen werden können.

Werden jedoch wie im erfindungsgemäßen Verfahren gemäß Schritt b) nur einzelne galvanisch katalytisch wirksame Keime auf der Oberfläche der Folie erzeugt oder auf die Oberfläche der Folie aufgebracht, dann ändert sich zwar durch die Verstreckung der Folie die Flächendichte der Keime, eine Rissbildung ist aber ausgeschlossen.

Weiterhin wird die Art der Modifizierung der Oberfläche der Folie in Schritt b) so gewählt, dass die galvanisch katalytisch wirksamen Keime ihre katalytischen Eigenschaften während des Umformens nicht verlieren. Hierfür eignen sich insbesondere Atome, Moleküle, Cluster oder metallische Partikel, vorzugsweise aus Edelmetallen, die entweder auf der Oberfläche physikalisch haften bleiben oder kovalent an die Oberfläche gebunden sind.

Im letzten Schritt d) wird der gemäß Schritt c) hergestellte Formkörper mit einen außenstromlosen Elektrolyten in Kontakt gebracht, wodurch die galvanisch katalytisch wirksamen Keime jeweils innerhalb der gewünschten Bereiche flächig zu einer vorzugsweise elektrisch leitfähigen, metallischen Schicht geschlossen werden. Für die außenstromlose Abscheidung eignen sich je nach Anwendung die aus der Galvanik bekannten Metalle wie z.B. Nickel, Kupfer, Gold, Platin oder Palladium. Bei der Auswahl des abzuscheidenden Metalls muss jedoch eine entsprechende Abstimmung der katalytischen Eigenschaften der Keime mit dem Elektrolyten erfolgen, um Abscheidung und Schichtbildung zu ermöglichen.

Das erfindungsgemäße Verfahren bietet entscheidende Vorteile gegenüber den bekannten MID-Verfahren. Die Vorstrukturierung auf einer Folie ermöglicht eine Strukturierung mit deutlich höherer Auflösung im Vergleich zu den bisherigen Verfahren.

Ein weiterer Vorteil besteht darin, dass die Geometrie des Formkörpers im Wesentlichen nur durch das Thermoformverfahren selbst, aber nicht durch die Strukturierung der Leiterbahnen eingeschränkt ist. Daher können auch bei starker Verstreckung komplexe dreidimensionale Mikrostrukturen mit Leiterbahnen versehen werden, solange die Struktur selbst durch ein Thermoformverfahren herstellbar ist. Das erfindungsgemäße Verfahren erlaubt es, dreidimensionale allseitig dünnwandige Mikrostrukturen mit Leiterbahnen zu versehen, was mit anderen mikrostrukturtechnischen Verfahren kaum oder nicht möglich ist.

Insbesondere in Anwendung, bei denen mit dem erfindungsgäßen verfahren hergestellte Leiterbahnen als Heizelemente oder zum Ein- oder Auskoppeln von elektromagnetischen Feldern in die Mikrostruktur dienen soll, ist es vorteilhaft, wenn dies nur durch extrem dünne Folien hindurch erfolgen muss. Damit ist das Beheizen des Inhaltes einer thermogeformten Mikrokavität, die z. B. zur Aufbewahrung von anorganischen oder organischen Molekülen, von Biomolekülen, prokaryontischen oder eukaryontischen Zellen oder Geweben oder als Zellcontainer zur Kultivierung oder Expansion von prokaryontischen oder eukaryontischen Zellen, als Biosensor oder als Bioreaktor dient, durch einen auf der Außenfläche befindlichen Heizleiter bei geringerem Energieaufwand schneller möglich, da die wärmeisolierende Kunststofffolie in diesem Bereich extrem dünn ist.

Die Erfindung wird im Folgenden anhand eines Ausführungsbeispiels näher erläutert.

Hierzu wurde als Substrat eine 50 µm dicke Polycarbonatfolie mit einer einseitigen statistischen Oberflächenrauhigkeit bereitgestellt. Auf der aufgerauten Seite der Folie wurden mittels PVD Goldkeime mit einer Schlitzblende (Schlitzbreite ca. 50 µm) in den gewünschten Bereichen auf der Oberfläche der Folie verankert (Bekeimung der Folie).

Danach wurde die mit Keimen im Bereich des Schlitzes versehene (bekeimte) Folie zu einem dreidimensionalen Formkörper thermogeformt, so dass die raue und teilweise aktivierte Oberfläche die innere Oberfläche des Formkörpers bildete. Die Folie wurde hierzu in zylindrische Mikrokavitäten mit Durchmessern von 350 µm eines Formwerkzeuges 150 µm tief eingeformt. Durch den Verstreckprozess erfolgt eine Ausdünnung der Wanddicke, die dann teilweise erheblich weniger als 50 µm dick war.

Anschließend wurde der in Teilbereichen auf seiner Oberfläche bekeimte Formkörper komplett in einen außenstromlosen autokatalytischen Gold-Elektrolyten gegeben, wobei darauf geachtet wurde dass eine vollständige Benetzung der Oberfläche erfolgte. Nach Entnahme wurde der Formkörper gespült und getrocknet. Eine durchgehende elektrische Leitfähigkeit konnte über mehrere Mikrokavitäten hinweg nachgewiesen werden. Die Breite der Leiterbahn betrug ca. 80 µm, ihre Länge ca. 20 mm.

## Patentansprüche

1. Verfahren zur Herstellung einer Schicht auf einem Formkörper mit den Schritten
a) Bereitstellen einer umformbaren Folie,
b) Anschließendes Aufbringen oder Erzeugen von galvanisch katalytisch wirksamen Keimen, die innerhalb derjenigen Bereiche auf der Oberfläche der bereitgestellten Folie, die für die Schicht vorgesehen sind, verankert sind,
c) Umformen der in ausgewählten Bereichen mit Keimen versehenen Folie zu einem Formkörper,
d) Galvanische Abscheidung auf der Oberfläche des gemäß Schritt c) hergestellten Formkörpers, wodurch die Keime zu der Schicht verbunden werden.

2. Verfahren nach Anspruch 1 mit einzelnen Atomen, Molekülen, Clustern oder Partikeln als Keime.

3. Verfahren nach Anspruch 1 oder 2 mit Keimen, die metallisch leitende Eigenschaften aufweisen.

4. Verfahren nach Anspruch 3 mit Keimen, die ein Edelmetall enthalten.

5. Verfahren nach Anspruch 3 oder 4 mit Keimen, die eine metallorganische Verbindung enthalten.

6. Verfahren nach einem der Ansprüche 1 bis 5 mit Keimen, die autokatalytische, ionogene oder kolloidale Eigenschaften aufweisen.

7. Verfahren nach einem der Ansprüche 1 bis 6, wobei die Keime mittels Abscheidung auf die Oberfläche der Folie aufgebracht werden.

8. Verfahren nach einem der Ansprüche 1 bis 7, wobei die Keime mittels Licht- oder Teilchenstrahlung auf der Oberfläche der Folie erzeugt werden.

9. Verfahren nach einem der Ansprüche 1 bis 8 mit einer Folie aus Kunststoff.

10. Verfahren nach Anspruch 9 mit einer Folie aus einem thermo- , plastischen Kunststoff.

11. Verfahren nach Anspruch 9 oder 10, wobei die Folie für das Umformen erwärmt wird.

12. Verfahren nach einem der Ansprüche 9 bis 11, wobei die Folie beim Umformen thermogeformt oder tiefgezogen wird.

13. Verfahren nach einem der Ansprüche 9 bis 12 mit einer Folie, die eine Dicke von 1 µm bis 1000 µm aufweist.

14. Verfahren nach einem der Ansprüche 1 bis 13, wobei Nickel, Kupfer, Gold, Platin oder Palladium galvanisch auf der Oberfläche des Formkörpers abgeschieden werden.

## Claims

1. Method for producing a layer on a moulding, with the following steps:
a) Preparation of a deformable film,
b) Subsequent application or generation of galvanic catalytic effective nuclei which are anchored within those regions on the surface of the prepared film which are provided for the layer,
c) Forming of the film provided with nuclei in selected regions into a moulding,
d) Electrodeposition on the surface of the moulding produced in accordance with step c), as a result of which the nuclei become combined to form the layer.

2. Method according to Claim 1 with individual atoms, molecules, clusters or particles as nuclei.

3. Method according to Claim 1 or 2_with nuclei which have metallic conducting characteristics.

4. Method according to Claim 3 with nuclei which include a precious metal.

5. Method according to Claim 3 or 4 with nuclei which include a metallo-organic compound.

6. Method according to one of Claims 1 to 5 with nuclei which have autocatalytic, ionogenic or colloidal characteristics.

7. Method according to one of Claims 1 to 6, wherein the nuclei are applied to the surface of the film by means of precipitation.

8. Method according to one of Claims 1 to 7, wherein the nuclei are generated on the surface of the film by means of luminous radiation or particle radiation.

9. Method according to one of Claims 1 to 8, with a film made from plastics material.

10. Method according to Claim 9, with a film made from a thermoplastic plastics material.

11. Method according to Claim 9 or 10, wherein the film is heated for forming.

12. Method according to one of Claims 9 to 11, wherein the film is thermoformed or deep-drawn during forming.

13. Method according to one of Claims 9 to 12, with a film which has a thickness of 1 µm to 1,000 µm.

14. Method according to one of Claims 1 to 13, wherein nickel, copper, gold, platinum or palladium are electrodeposited on the surface of the moulding.

## Revendications

1. Procédé de réalisation d'une couche sur un corps de forme comprenant les étapes suivantes :
a) on réalise un film déformable,
b) ensuite on applique ou on réalise des germes à action catalytique galvanique, qui sont accrochés dans la zone concernée à la surface du film fourni, prévu pour la couche,
c) on transforme le film dans des zones choisies qui comportent des germes pour obtenir un corps moulé,
d) on fait un dépôt galvanique à la surface du corps moulé réalisé selon l'étape c), si bien que les germes se réunissent sous la forme d'une couche.

2. Procédé selon la revendication 1,
comportant comme germes des atomes, des molécules, des clusters, ou des particules, séparés.

3. Procédé selon la revendication 1 ou 2,
utilisant des germes qui ont des propriétés métalliques conductrices.

4. Procédé selon la revendication 3,
comprenant des germes qui contiennent un métal noble.

5. Procédé selon les revendications 3 ou 4,
concernant des germes qui contiennent une combinaison organométallique.

6. Procédé selon l'une des revendications 1 à 5,
pour des germes qui ont des propriétés d'autocatalyse, des propriétés ionogènes ou des propriétés colloïdales.

7. Procédé selon l'une des revendications 1 à 6,
**caractérisé en ce que**
les germes sont appliqués par dépôt sur la surface du film.

8. Procédé selon l'une des revendications 1 à 7,
selon lequel les germes sont réalisés à la surface du film par un rayonnement optique ou un rayonnement de particules.

9. Procédé selon l'une des revendications 1 à 8,
pour un film en matière plastique.

10. Procédé selon la revendication 9,
comportant un film en matière thermoplastique.

11. Procédé selon la revendication 9 ou la revendication 10,
selon lequel le film est chauffé pour être transformé.

12. Procédé selon l'une des revendications 9 à 11,
selon lequel le film est transformé de manière thermogène ou par un emboutissage profond.

13. Procédé selon l'une des revendications 9 à 12,
concernant un film d'une épaisseur de 1 µm à 1 000 µm.

14. Procédé selon l'une des revendications 1 à 13,
selon lequel on dépose du nickel, cuivre, or, platine ou palladium par voie galvanique à la surface de la pièce de forme.
